# EUROPEAN PATENT APPLICATION

(11) **EP 3 545 954 A1**
(43) Date of publication of application: **02.10.2019**
(21) Application number: 17874965.1
(22) Date of filing: 27.11.2017
(51) Int. Cl.: A61K 31/427, A61P 3/10, A61P 13/12

(54) **THERAPEUTIC DRUG OR PROPHYLACTIC DRUG FOR DIABETIC NEPHROPATHY**

(30) Priority: 28.11.2016 JP 2016229972
(71) Applicant: Teijin Pharma Limited, Tokyo 100-0013 (JP)
(72) Inventor: SHIRAKURA, Takashi, Tokyo 100-0013 (JP); TSUJIMOTO, Shunsuke, Tokyo 100-0013 (JP); TSUBOSAKA, Yoshiki, Tokyo 100-0013 (JP)
(74) Representative: Cockerton, Bruce Roger
(86) International application number: PCT/JP2017/042429
(87) International publication number: WO 2018/097294

(57) **Abstract**

The present invention pertains to a therapeutic drug or prophylactic drug for diabetic nephropathy, said drug comprising a compound represented by formula (I) or a pharmaceutically acceptable salt thereof as an active ingredient.

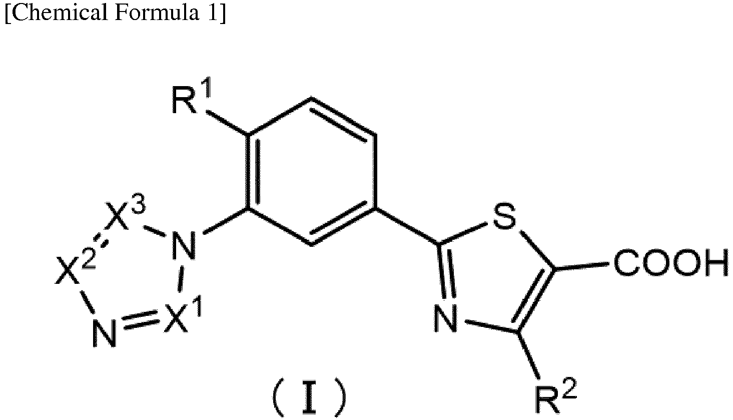

## Description

### FIELD

The present invention relates to a therapeutic drug or prophylactic drug for diabetic nephropathy.

### BACKGROUND

Diabetic nephropathy is one of the three major complications of diabetes, and the number of patients continues to increase throughout the world. In Japan as well, there has been ever increasing incidence in recent years, and it has become the major causative condition leading to new artificial dialysis cases. The prognosis for dialysis patients is poor, and therefore treatment methods are desired to inhibit progression of diabetic nephropathy or to improve diabetic nephropathy. Treatment of diabetic nephropathy is centered on blood sugar level management, dietary restriction and blood pressure management, while the only therapeutic agents with efficacy for diabetic nephropathy that are used are a certain subset of renin-angiotensin inhibitors.

Clinical findings of diabetic nephropathy include changes in glomerular filtration rate (GFR) commonly seen with chronic kidney disease, as well as albuminuria and proteinuria, and the urinary albumin or protein level, and also the GFR, are used in Japan as well for classification of diabetic nephropathy.

Xanthine oxidase is an enzyme that catalyzes conversion of hypoxanthine to xanthine, and further to uric acid, during the course of nucleic acid metabolism. Xanthine oxidase inhibitors are used in clinical as therapeutic agents for gout and hyperuricemia. Several xanthine oxidase inhibitors such as topiraxostat, allopurinol and febuxostat are considered effective for diabetic nephropathy (PTL 1, NPLs 1, 2 and 3). Azolebenzene derivatives are also known as xanthine oxidase inhibitors, in addition to these compounds (PTL 2). Hyperuricemia is defined as a serum uric acid level exceeding 7.0 mg/dL, and for lasting improvement it is considered more beneficial for it to be reduced to 6 mg/dL and sometimes to 5 mg/dL (NPLs 4 and 5).

One known cause of diabetic nephropathy is the continuous prolonged condition of high blood glucose level in diabetes, and resulting progression of systemic arteriosclerosis that leads to breakdown of small blood vessels in the glomeruli of the kidney where capillaries are abundant, and breakdown and clogging of the capillary network structure, thus interfering with filtration of waste products. It has been reported that uric acid produced by xanthine oxidase (XO) leads to renal disorder by causing tubular damage in the kidneys, and that XO inhibitors have the potential to ameliorate diabetic nephropathy, primarily by reducing the action of uric acid and alleviating such renal tubular damage (NPLs 6 and 7). On the other hand, albuminuria and proteinuria which are characteristic of diabetic nephropathy are known to be caused by glomerular injury in kidneys.

### [CITATION LIST]

### [PATENT LITERATURE]

[PTL 1] WO2012/060308
[PTL 2] WO2014/119681

### [NON PATENT LITERATURE]

[NPL 1] Iranian Journal of Kidney Disease 2010, 4(2). pp128-132
[NPL 2] Nephron Experimental Nephrology 2012,121. pp109-121
[NPL 3] American Journal of Nephrology 2014, 40. pp56-63
[NPL 4] Arthritis Care & Research Vol.64, No.10, October 2012, pp1431-1446
[NPL 5] Ann Rheum Dis 2016; 0:1-14
[NPL 6] American Journal of Physiology Renal Physiology 2009: 297:pp.481-488
[NPL 7] Nephron Experimental Physiology 2012: 121:pp.109-121

### SUMMARY

### [TECHNICAL PROBLEM]

The problem to be solved by the present invention is that of providing a therapeutic drug or prophylactic drug for diabetic nephropathy.

### [SOLUTION TO PROBLEM]

As a result of ardent research, the present inventors have completed this invention upon finding that compounds represented by the following formula (I): 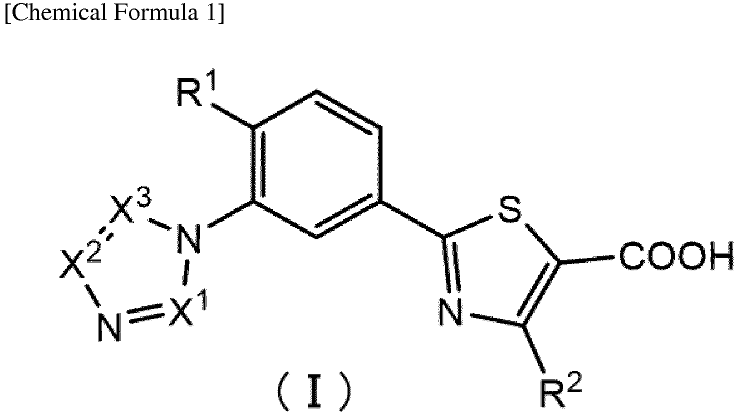 namely, azolebenzene derivatives having a benzene structure with three substituents, having a 2-thiazole ring at the 1-position and having a 1,3-nitrogen-containing azole ring at the 3-position, are useful for treating or preventing diabetic nephropathy.

Specifically, the invention provides the following.
[1] A therapeutic drug or prophylactic drug for diabetic nephropathy containing a compound represented by formula (I) or its pharmaceutically acceptable salt as an active ingredient. 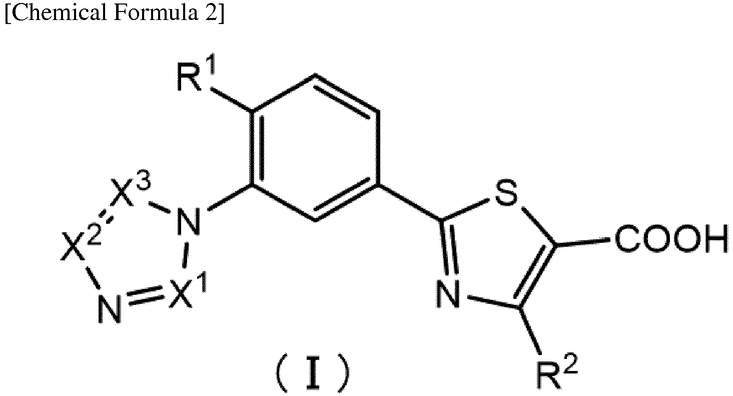 [wherein,
   R¹ represents OR, NRR' that optionally forms a ring, or SR, where R and R' each independently represent a hydrogen atom, an alkyl group of 1 to 8 carbon atoms optionally substituted with one or more alkoxy groups of 1 to 8 carbon atoms, halogen atoms or hydroxyl groups, an aryl group optionally substituted with one or more alkyl groups of 1 to 8 carbon atoms, alkoxy groups of 1 to 8 carbon atoms or halogen atoms, or a heteroaryl group optionally substituted with one or more alkyl groups of 1 to 8 carbon atoms, alkoxy groups of 1 to 8 carbon atoms or halogen atoms.
   R² represents a hydrogen atom or an alkyl group of 1 to 8 carbon atoms.
   X¹ , X² and X³ are each independently CR³ or a nitrogen atom, or X¹ is CR³ or a nitrogen atom, and X² and X³ together form a benzene ring. R³ represents a hydrogen atom or an alkyl group of 1 to 8 carbon atoms.]
[2] The therapeutic drug or prophylactic drug according to [1], wherein R¹ is OR.
[3] The therapeutic drug or prophylactic drug according to [1], wherein R¹ is SR.
[4] The therapeutic drug or prophylactic drug according to [1], wherein R¹ is NRR' that optionally forms a ring.
[5] The therapeutic drug or prophylactic drug according to any one of [1] to [4], wherein R and R' are each independently an alkyl group of 1 to 8 carbon atoms optionally substituted with one or more alkoxy groups of 1 to 8 carbon atoms, halogen atoms or hydroxyl groups, or an aryl group optionally substituted with one or more alkyl groups of 1 to 8 carbon atoms, alkoxy groups of 1 to 8 carbon atoms or halogen atoms.
[6] The therapeutic drug or prophylactic drug according to [5], wherein R and R' are each independently an alkyl group of 1 to 8 carbon atoms optionally substituted with one or more alkoxy groups of 1 to 8 carbon atoms, halogen atoms or hydroxyl groups.
[7] The therapeutic drug or prophylactic drug according to [6], wherein R¹ is OR or SR, and R is an isopropyl, isobutyl or neopentyl group.
[8] The therapeutic drug or prophylactic drug according to any one of [1] to [7], wherein R² is a hydrogen atom or an alkyl group of 1 to 3 carbon atoms.
[9] The therapeutic drug or prophylactic drug according to [8], wherein R² is a methyl group.
[10] The therapeutic drug or prophylactic drug according to any one of [1] to [9], wherein X¹, X² and X³ are each independently CR³ or a nitrogen atom.
[11] The therapeutic drug or prophylactic drug according to [10], wherein X¹ is a nitrogen atom, X² is CR³ or a nitrogen atom, and X³ is CR³.
[12] The therapeutic drug or prophylactic drug according to any one of [1] to [11], wherein R³ is hydrogen.
[13] The therapeutic drug or prophylactic drug according to [1], wherein the compound represented by formula (I) is a compound selected from the following group, or a pharmaceutically acceptable salt of the same. 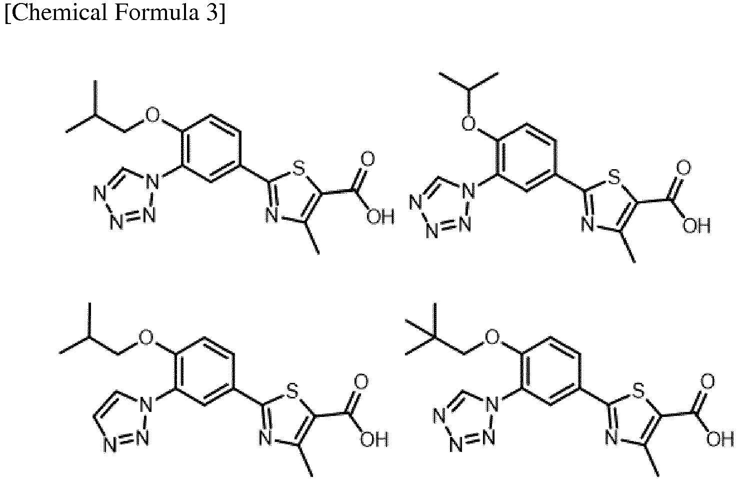
[14] The therapeutic drug or prophylactic drug according to any one of [1] to [13], wherein the diabetic nephropathy is type II diabetic nephropathy.

### [ADVANTAGEOUS EFFECTS OF INVENTION]

A compound of formula (I) or its pharmaceutically acceptable salt to be used for the invention is useful as a therapeutic drug or prophylactic drug for diabetic nephropathy.

### DESCRIPTION OF EMBODIMENTS

The technical terms used either alone or in combination throughout the present specification will now be explained. Unless otherwise specified, the explanations of the substituents apply for all positions. When variables are present for a given constituent element, the definitions independently apply to each constituent element. Any combinations of substituents and variables are possible so long as the combinations yield chemically stable compounds.

According to the invention, "halogen atom" means fluorine, chlorine, bromine or iodine.

Also, for the purpose of the invention, "alkyl group" means a monovalent saturated straight-chain, cyclic or branched aliphatic hydrocarbon group, and examples of "alkyl groups of 1 to 8 carbon atoms" include methyl, ethyl, n-propyl, n-butyl, n-pentyl, n-hexyl, isopropyl, isobutyl, s-butyl, t-butyl, isopentyl, 2-methylbutyl, neopentyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, 2-ethylbutyl, t-pentyl, isohexyl, cyclopropyl, cyclobutyl, cyclopentyl, cyclohexyl, cycloheptyl, cyclopropylmethyl, cyclobutylmethyl, cyclopentylmethyl, cyclohexylmethyl and cycloheptylmethyl groups. The term "alkyl group of 1 to 3 carbon atoms" includes methyl, ethyl, n-propyl and isopropyl groups.

According to the invention, "alkoxy group" means a monovalent saturated straight-chain, cyclic or branched aliphatic hydrocarbon oxy group. Examples of "alkoxy groups of 1 to 8 carbon atoms" include methoxy, ethoxy, n-propoxy, n-butoxy, n-pentyloxy, n-hexyloxy, isopropoxy, isobutoxy, s-butoxy, t-butoxy, isopentyloxy, 2-methylbutoxy, neopentyloxy, cyclopropoxy, cyclobutoxy, cyclopentyloxy, cyclohexyloxy, cycloheptyloxy, cyclopropylmethoxy, cyclobutylmethoxy, cyclopentylmethoxy and cyclohexylmethoxy groups.

According to the invention, "aryl group" means a monocyclic or bicyclic aromatic hydrocarbon group of 6 to 10 carbon atoms. Aryl groups include phenyl, naphthyl, indenyl, tetrahydronaphthyl, indanyl and azulenyl groups.

According to the invention, "heteroaryl group" means a monocyclic or bicyclic aromatic heterocyclic group having 1 to 5 heteroatoms selected from among oxygen atoms, sulfur atoms and nitrogen atoms. Heteroaryl groups include pyridyl, pyrazyl, pyrimidyl, furyl, thienyl, isooxazolyl, isothiazolyl, benzofuranyl, benzothienyl, benzothiazolyl, benzoimidazolyl, benzooxazolyl, pyranyl, imidazolyl, oxazolyl, thiazolyl, triazinyl, triazolyl, benzooxazolyl and benzoisooxazolyl groups.

According to the invention, an "optionally substituted alkyl group of 1 to 8 carbon atoms" is an alkyl group of 1 to 8 carbon atoms that optionally has one or more substituents at substitutable positions. Substituents for the alkyl group of 1 to 8 carbon atoms include alkoxy groups of 1 to 8 carbon atoms, halogen atoms and hydroxyl groups. When multiple substituents are present, the substituents may be the same or different.

According to the invention, an "optionally substituted aryl group" is an aryl group that optionally has one or more substituents at substitutable positions. Substituents for the aryl group include alkyl groups of 1 to 8 carbon atoms, alkoxy groups of 1 to 8 carbon atoms and halogen atoms. When multiple substituents are present, the substituents may be the same or different.

According to the invention, an "optionally substituted heteroaryl group" is a heteroaryl group of that optionally has one or more substituents at substitutable positions. Substituents for the heteroaryl group include alkyl groups of 1 to 8 carbon atoms, alkoxy groups of 1 to 8 carbon atoms and halogen atoms. When multiple substituents are present, the substituents may be the same or different.

In formula (I), R¹ represents OR, NRR' that optionally forms a ring, or SR. R and R' each independently represent a hydrogen atom, an alkyl group of 1 to 8 carbon atoms optionally substituted with one or more alkoxy groups of 1 to 8 carbon atoms, halogen atoms or hydroxyl groups, an aryl group optionally substituted with one or more alkyl groups of 1 to 8 carbon atoms, alkoxy groups of 1 to 8 carbon atoms or halogen atoms, or a heteroaryl group optionally substituted with one or more alkyl groups of 1 to 8 carbon atoms, alkoxy groups of 1 to 8 carbon atoms or halogen atoms. R¹ is preferably OR. When R¹ is OR or SR, R is preferably an alkyl group of 1 to 8 carbon atoms optionally substituted with one or more alkoxy groups of 1 to 8 carbon atoms, halogen atoms or hydroxyl groups, or an aryl group optionally substituted with one or more alkyl groups of 1 to 8 carbon atoms, alkoxy groups of 1 to 8 carbon atoms or halogen atoms. More preferably, it is an alkyl group of 1 to 8 carbon atoms optionally substituted with one or more alkoxy groups of 1 to 8 carbon atoms or hydroxyl groups. Most preferably it is an isopropyl, isobutyl or neopentyl group. When R¹ is NRR' that optionally forms a ring, "NRR' forms a ring" means that R and R', together with their bonded nitrogen atoms, form a nitrogen-containing saturated ring. When R¹ is an NRR' that optionally forms a ring, R and R' are each independently preferably an alkyl group of 1 to 8 carbon atoms optionally substituted with a hydroxyl group, R and R' are each independently a methyl, ethyl or isopropyl group, or R and R' more preferably, together with their bonded nitrogen atoms, form a pyrrolidin-1-yl, piperidin-1-yl or morpholin-1-yl group.

In formula (I), R² represents a hydrogen atom or an alkyl group of 1 to 8 carbon atoms. Specific examples of "the alkyl group of 1 to 8 carbon atoms" are as defined above. It is preferably a hydrogen atom or an alkyl group of 1 to 3 carbon atoms, and specifically a methyl, ethyl, n-propyl or isopropyl group. It is more preferably a hydrogen atom or a methyl group. It is most preferably a methyl group.

In formula (I), X¹ , X² and X³ are each independently CR³ or a nitrogen atom, or X¹ is CR³ or a nitrogen atom and X² and X³ together form a benzene ring. R³ represents a hydrogen atom or an alkyl group of 1 to 8 carbon atoms. When X¹ is CR³ or a nitrogen atom and X² and X³ together form a benzene ring, it may be represented by the following structural formula.

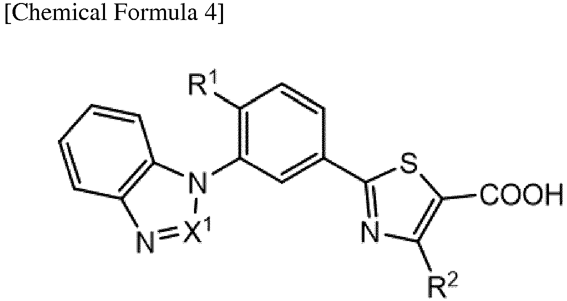

Preferably, X¹, X² and X³ are independently CR³ or a nitrogen atom. A more preferred combination is one in which X¹ is a nitrogen atom, X² is CR³ or a nitrogen atom and X³ is CR³. For all combinations, R³ is preferably hydrogen. When X¹ is a nitrogen atom, X² is CH or a nitrogen atom and X³ is CH, it may be represented by the following structural formula.

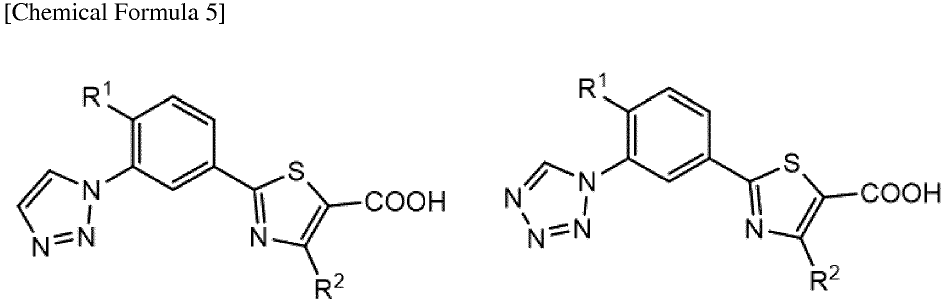

When R¹ in formula (I) is OR, NRR' that optionally forms a ring or SR, preferably the combination of R, R', R², X¹, X² and X³ is such that each of them are the preferred groups mentioned above, and R³ is a hydrogen atom or an alkyl group of 1 to 8 carbon atoms. In the preferred combination of R, R', R², X¹, X² and X³, R³ is more preferably a hydrogen atom.

A more preferred combination of R, R', R², X¹, X² and X³ is one which is a combination of the more preferred groups, and R³ is a hydrogen atom or an alkyl group of 1 to 8 carbon atoms. In such a more preferred combination of R, R', R², X¹ X² and X³, R³ is more preferably a hydrogen atom.

An even more preferred combination of R, R', R², X¹, X² and X³ is one in which R is an isopropyl, isobutyl or neopentyl group, R² is a methyl group, X¹ is a nitrogen atom, X² is CR³ or a nitrogen atom and X³ is CR³, and R³ is a hydrogen atom.

In these preferred, more preferred and even more preferred combinations of R, R', R², X¹, X² and X³, R¹ is preferably OR.

Specific examples of the preferred combinations of R¹, R, R', R², X¹, X² and X³ for formula (I) of the invention are listed below as combinations 1) to 9).
1) R¹ is OR; R is an alkyl group of 1 to 8 carbon atoms optionally substituted with one or more alkoxy groups of 1 to 8 carbon atoms, halogen atoms or hydroxyl groups, or an aryl group optionally substituted with one or more alkyl groups of 1 to 8 carbon atoms, alkoxy groups of 1 to 8 carbon atoms or halogen atoms; R² is a hydrogen atom or an alkyl group of 1 to 3 carbon atoms; X¹ is a nitrogen atom; X² is CR³ or a nitrogen atom; X³ is CR³; and R³ is a hydrogen atom.
2) R¹ is OR; R is an alkyl group of 1 to 8 carbon atoms optionally substituted with one or more alkoxy groups of 1 to 8 carbon atoms or hydroxyl groups; R² is a hydrogen atom or an alkyl group of 1 to 3 carbon atoms; X¹ is a nitrogen atom; X² is CR³ or a nitrogen atom; X³ is CR³; and R³ is a hydrogen atom.
3) R¹ is OR; R is an isopropyl, isobutyl or neopentyl group; R² is a hydrogen atom or a methyl group; X¹ is a nitrogen atom; X² is CR³ or a nitrogen atom; X³ is CR³; and R³ is a hydrogen atom.
4) R¹ is SR; R is an alkyl group of 1 to 8 carbon atoms optionally substituted with one or more alkoxy groups of 1 to 8 carbon atoms, halogen atoms or hydroxyl groups, or an aryl group optionally substituted with one or more alkyl groups of 1 to 8 carbon atoms, alkoxy groups of 1 to 8 carbon atoms or halogen atoms; R² is a hydrogen atom or an alkyl group of 1 to 3 carbon atoms; X¹ is a nitrogen atom; X² is CR³ or a nitrogen atom; X³ is CR³; and R³ is a hydrogen atom.
5) R¹ is SR; R is an alkyl group of 1 to 8 carbon atoms optionally substituted with one or more alkoxy groups of 1 to 8 carbon atoms or hydroxyl groups; R² is a hydrogen atom or an alkyl group of 1 to 3 carbon atoms; X¹ is a nitrogen atom; X² is CR³ or a nitrogen atom; X³ is CR³; and R³ is a hydrogen atom.
6) R¹ is SR; R is an isopropyl, isobutyl or neopentyl group; R² is a hydrogen atom or a methyl group; X¹ is a nitrogen atom; X² is CR³ or a nitrogen atom; X³ is CR³; and R³ is a hydrogen atom.
7) R¹ is NRR' that optionally forms a ring; R and R' are each independently an alkyl group of 1 to 8 carbon atoms optionally substituted with one or more alkoxy groups of 1 to 8 carbon atoms or hydroxyl groups; R² is a hydrogen atom or an alkyl group of 1 to 3 carbon atoms; X¹ is a nitrogen atom; X² is CR³ or a nitrogen atom; X³ is CR³; and R³ is a hydrogen atom.
8) R¹ is NRR' that optionally forms a ring; R and R' are each independently a methyl, ethyl or isopropyl group, or R and R' together with their bonding nitrogen atom, form a pyrrolidin-1-yl, piperidin-1-yl or morpholin-1-yl group; R² is a hydrogen atom or an alkyl group of 1 to 3 carbon atoms; X¹ is a nitrogen atom; X² is CR³ or a nitrogen atom; X³ is CR³; and R³ is a hydrogen atom.
9) R¹ is NRR' that optionally forms a ring; R and R' are each independently a methyl, ethyl or isopropyl group, or R and R' together with their bonding nitrogen atom, form a pyrrolidin-1-yl, piperidin-1-yl or morpholin-1-yl group; R² is a hydrogen atom or methyl group; X¹ is a nitrogen atom; X² is CR³ or a nitrogen atom; X³ is CR³; and R³ is a hydrogen atom.

The compounds of the invention are compounds exhibiting excellent xanthine oxidase inhibiting activity. The compounds of the invention also have excellent activity of lowering uric acid. The compounds of the invention still further have long-lasting effects of lowering uric acid for prolonged periods.

Specific compounds of formula (I) include the compounds mentioned in WO2014/119681. Preferred compounds are those listed in the following table.

**[Chemical Formula 6]**

| Compound | Compound name | Structure |
|---|---|---|
| 1 | 4-Methyl-2-[4-(2-methylpropoxy)-3-(1H-1,2,3,4-tetrazol-1-yl)phenyl]-1,3-thiazole-5-carboxylic acid | 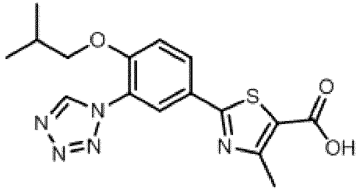 |
| 2 | 4-Methyl-2-[4-(propan-2-yloxy)-3-(1H-1,2,3,4-tetrazol-1-yl)phenyl]-1,3-thiazole-5-carboxylic acid | 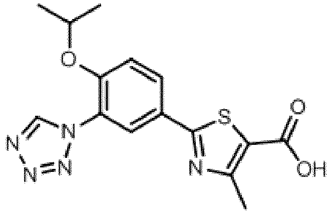 |
| 3 | 4-Methyl-2-[4-(2-methylpropoxy)-3-(1H-1,2,3-triazol-1-yl)phenyl]-1,3-thiazole-5-carboxylic acid | 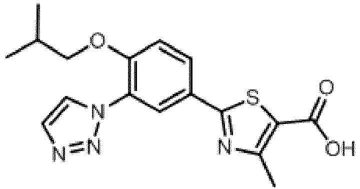 |
| 4 | 2-[4-(2,2-Dimethylpropoxy)-3-(1H-1,2,3,4-tetrazol-1-yl)phenyl]-4-methyl-1,3-thiazole-5-carboxylic acid | 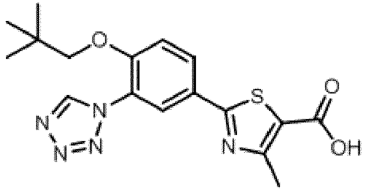 |

The compounds of formula (I) of the invention can be produced by a publicly known method, such as the method described in WO2014/119681. Of the compounds represented by formula (I), preferred compounds and their pharmaceutically acceptable salts are not particularly restricted so long as they are pharmaceutically acceptable salts, examples of such salts including salts of inorganic acids such as hydrochloric acid, hydrobromic acid, sulfuric acid, nitric acid, phosphoric acid and carbonic acid; salts of organic acids such as maleic acid, fumaric acid, citric acid, malic acid, tartaric acid, lactic acid, succinic acid, benzoic acid, oxalic acid, methanesulfonic acid, benzenesulfonic acid, p-toluenesulfonic acid, acetic acid, trifluoroacetic acid and formic acid; salts of amino acids such as glycine, lysine, arginine, histidine, ornithine, glutamic acid and aspartic acid; salts of alkali metals such as sodium, potassium and lithium; salts of alkaline earth metals such as calcium and magnesium; salts of metals such as aluminum, zinc and iron; salts of organic onium compounds such as tetramethylammonium and choline; and salts of organic bases such as ammonia, propanediamine, pyrrolidine, piperidine, pyridine, ethanolamine, N,N-dimethylethanolamine, 4-hydroxypiperidine, t-octylamine, dibenzylamine, morpholine, glucosamine, phenylglycylalkyl ester, ethylenediamine, N-methylglucamine, guanidine, diethylamine, triethylamine, dicyclohexylamine, N,N'-dibenzylethylenediamine, chloroprocaine, procaine, diethanolamine, N-benzylphenylamine, piperazine and tris(hydroxymethyl)aminomethane.

Compounds represented by formula (I) and their salts also include various hydrates and solvates. Solvents for solvates are not particularly restricted, and include methanol, ethanol, 1-propanol, 2-propanol, butanol, t-butanol, acetonitrile, acetone, methyl ethyl ketone, chloroform, ethyl acetate, diethyl ether, t-butylmethyl ether, tetrahydrofuran (THF), 1,4-dioxane, 1,2-dimethoxyethane, 1,2-diethoxyethane, benzene, toluene, dimethylformamide (DMF) and dimethyl sulfoxide (DMSO).

The compounds of the invention also include stereoisomers, racemic mixtures and all possible optically active forms of the compounds represented by formula (I) and their salts.

According to the invention, the compounds represented by formula (I) and their pharmaceutically acceptable salts may be used as drugs for treatment or prevention of diabetic nephropathy (also known as "diabetic kidney disease"), which are clinically applicable as xanthine oxidase inhibitors. Moreover, the invention includes a treatment method and prophylactic method for diabetic nephropathy, by administration of a compound represented by formula (I) or its pharmaceutically acceptable salt. Specific forms of diabetic nephropathy include diabetic nephropathy caused by type I or type II diabetes, and preferably diabetic nephropathy caused by type II diabetes ("type II diabetic nephropathy", or "diabetic nephropathy in type II diabetes", "diabetic nephropathy accompanying type II diabetes"). Since a compound represented by formula (I) or its pharmaceutically acceptable salt is useful for diabetic nephropathy elicited by glomerular damage, it may be considered useful for diabetic glomerulosclerosis as well, as a type of diabetic nephropathy.

According to the invention, "prophylactic" means prevention before incidence or onset in an individual without incidence or onset, and "therapeutic" means curing, suppression or amelioration of disease or symptoms in an individual with existing incidence or onset.

A compound represented by formula (I) or its pharmaceutically acceptable salt may be formulated into a medical composition together with a pharmaceutically acceptable carrier and/or diluent. The medical composition may be molded into various dosage forms and administered either perorally or parenterally. Examples of parenteral administration include administration into a vein, hypodermic region, muscle, transdermal region or rectal region.

A formulation containing one or more compounds represented by formula (I) of the invention or their salts as active ingredients is prepared using a carrier or excipient, as well as other additives, commonly used in formulation. Carriers or excipients for formulation may be in either solid or liquid form, examples including lactose, magnesium stearate, starch, talc, gelatin, agar, pectin, gum arabic, olive oil, sesame oil, cacao butter, ethylene glycol and other commonly used substances. The administration may be in any form such as oral administration in the form of a tablet, pill, capsule, granules, powder or liquid, or parenteral administration in the form of an injection for intravenous injection or intramuscular injection, a suppository or a transdermal form.

Administration of the compound represented by formula (I) of the invention or its pharmaceutically acceptable salt will differ depending on the type of disease, the route of administration, and the symptoms, age, gender and body weight of the patient, but it may usually be in a range of 0.01 to 1000 mg per adult per day, either once or divided over several times. However, since the dose will vary depending on a variety of conditions, lower doses than those mentioned above may be sufficient, or doses exceeding these ranges may be necessary.

### EXAMPLES

The present invention will now be explained in greater detail by examples. However, it is to be understood that the invention is not limited in any way by these Examples.

### [Example 1]

To confirm the ameliorating effect of 4-methyl-2-[4-(2-methylpropoxy)-3-(1H-1,2,3,4-tetrazol-1-yl)phenyl]-1,3-thiazole-5-carboxylic acid (compound 1) on diabetic nephropathy, urinary albumin excretion, serum uric acid levels and renal XO activity in Zucker Diabetic Fatty (ZDF) rats (Charles River, Japan) were examineds. The ZDF rats had exhibited type II diabetes, and were therefore used as a diabetic nephropathy model.

Compound 1 (1 and 3 mg/kg) was orally administered to 8-week-old male ZDF rats once per day, for 13 weeks (1 and 3 mg/kg groups). As control groups, ZDF-Lean rats (Charles River, Japan) with normal blood glucose levels were orally administered a 0.5% methyl cellulose solvent once per day for 13 weeks (Control group), and ZDF rats were orally administered the solvent once per day for 13 weeks (Vehicle group). Urine was collected for 24 hours once every 2 weeks, from the start of administration until the 12th week of administration, and the urinary albumin concentration and urinary volume were measured for LBIS Rat Urinary Albumin Assay Kit (FUJIFILM Wako Shibayagi Corporation) by a Hitachi7180 autoanalyzer. The albumin excretion per day in the urine was calculated from the measured results. The urinary albumin excretion at each time point after initial administration and the cumulative urinary albumin excretion up to the 12th week after initial administration (AUC_{0-12wks}) were calculated, and the drug effect was examined. Administration was continued for 13 weeks from the initial administration, and the renal XO activity and blood plasma uric acid concentration were measured 24 hours after the final administration.

The renal XO activity was measured based on reaction producing isoxanthopterin, as a fluorescent substance resulting from oxidation of pterin by xanthine oxidoreductase. Kidneys were homogenized with a solution of potassium phosphate at pH 7.4 containing 1 mM EDTA and a protease inhibitor, to a tissue concentration of 5 mg/mL, and centrifuged for 15 minutes at 4°C, 12,000 rpm. The supernatant of the tissue homogenate was mixed with a solution containing 50 µM pterin and reacted at 37°C. As a control, reaction was conducted by the same method with a solution containing oxidase-type xanthine oxidoreductase (Calbiochem, buttermilk-derived) and 50 µM pterin. The fluorescence intensity of the produced isoxanthopterin was measured and adjusted based on the enzyme activity of the control and protein concentration, to calculate the XO activity.
The plasma uric acid level was measured by LC-MS/MS.
Compared to the Control group which had normal blood glucose levels, the urinary albumin excretion in the Vehicle group which consisted of ZDF rats that had type II diabetes and had been administered the solvent were increased in time-dependent manner. In the group administered compound 1, however, the urinary albumin excretion was significantly reduced compared to the Vehicle group, at both the 1 mg/kg and 3 mg/kg doses (Table 1 and Table 2).

Also, in the Vehicle group which consisted of ZDF rats that had diabetes and had been administered the solvent, the XO activity in the kidneys was significantly higher than the Control group which consisted of healthy animals. Since renal XO produces reactive oxygen species and uric acid which are thought to contribute to pathology in diabetic nephropathy, this suggests that increased renal XO activity contributes to onset and progression of diabetic nephropathy in ZDF rats. However, in the group administered compound 1, the 1 mg/kg and 3 mg/kg doses significantly inhibited renal XO activity compared to the Vehicle group, even at the 13th week after initial administration (24 hours after the final administration) (Table 3).

On the other hand, in the Vehicle group which consisted of ZDF rats that had diabetes and had been administered the solvent, the plasma uric acid concentration was significantly lower than the Control group which consisted of healthy animals. In contrast, in the group administered compound 1, the 1 mg/kg and 3 mg/kg doses significantly decreased the plasma uric acid concentration compared to the Vehicle group. (Table 4).

**[Table 1]**

| Urinary albumin excretion in each group before initial administration and at time points after initial administration | | | | |
|---|---|---|---|---|
| Administration period (weeks) | Urinary albumin excretion (µg/Day) | | | |
| | Group name | | | |
| | ** Control | Vehicle | Compound 1 1 mg/kg^{#} | Compound 1 3 mg/kg^{#} |
| 0 (before administration) | 0.45 ±0.09 | 3.47 ±0.70 | 3.37 ±0.83 | 3.91 ±1.06 |
| 2 | 0.43 ±0.20 | 9.53 ±2.39 | 4.78 ±0.87 | 7.64 ±1.66 |
| 4 | 0.26 ±0.04 | 17.77 ±3.98 | 8.90 ±1.63 | 14.43 ±2.90 |
| 6 | 0.63 ±0.36 | 35.15 ±3.32 | 21.96 ±3.79 | 21.97 ±1.75 |
| 8 | 0.62 ±0.40 | 51.26 ±6.52 | 38.74 ±5.64 | 23.38 ±3.09 |
| 10 | 0.38 ±0.14 | 77.71 ±9.26 | 41.33 ±5.47 | 37.37 ±4.11 |
| 12 | 0.32 ±0.07 | 102.99 ±12.84 | 60.00 ±8.58 | 65.88 ±8.33 |

| | | | | |
|---|---|---|---|---|
| (Values are mean ±SE) **p < 0.01: Significant difference (vs Vehicle group) (repeated measures ANOVA) ^{#}p < 0.05: Significant difference (vs Vehicle group) (time-dependent multiple comparison test) | | | | |

**[Table 2]**

| Cumulative urinary albumin excretion for each group from initial administration until 12th week after initial administration AUC_{0-12wks} | | | | |
|---|---|---|---|---|
| Group name | Control | Vehicle | Compound 1 1 mg/kg | Compound 1 3 mg/kg |
| Cumulative urinary albumin excretion (AUC_{0-12wks}) | 5.4±1.9** | 489.3±57.8 | 294.8±38.1^{#} | 279.4±27.1^{#} |

| | | | | |
|---|---|---|---|---|
| (Values are mean ±SE) **p < 0.01: Significant difference (vs Vehicle group) (Student t-test) #p < 0.05: Significant difference (vs Vehicle group) (Dunnett-type multiple comparison test) | | | | |

**[Table 3]**

| Renal XO activity of each group 24 hours after administration at 13th week after initial administration | | | | |
|---|---|---|---|---|
| Group name | Control | Vehicle | Compound 1 1 mg/kg | Compound 1 3 mg/kg |
| Renal XO activity (µU/mg protein) | 2913 ±261** | 6498 ±424 | 2238 ±417^{##} | 1187+334^{ff##} |

| | | | | |
|---|---|---|---|---|
| (Values are mean ±SE) **p < 0.01: Significant difference (vs Vehicle group) (Student t-test) ##p < 0.01: Significant difference (vs Vehicle group) (Dunnett-type multiple comparison test) | | | | |

**[Table 4]**

| Plasma uric acid concentration of each group 24 hours after administration at 13th week after initial administration | | | | |
|---|---|---|---|---|
| Group name | Control | Vehicle | Compound 1 1 mg/kg | Compound 1 3 mg/kg |
| Plasma uric acid concentration (mg/dL) | 0.46 ±0.06** | 0.31 ±0.04 | 0.16 ±0.01^{##} | 0.18 ±0.01^{##} |

| | | | | |
|---|---|---|---|---|
| (Values are mean ±SE) **p < 0.01: Significant difference (vs Vehicle group) (Student t-test) ##p < 0.01: Significant difference (vs Vehicle group) (Dunnett-type multiple comparison test) | | | | |

### [Example 2]

ZDF rats, a known animal model of diabetic nephropathy with type II diabetes, were used to examine the effect of compound 1 on urinary albumin excretion, as well as the effect on glomerular damage and renal tubular damage. Podocalyxyn was used as a marker for glomerular damage and Kidney Injury Marker-1 (KIM-1) was used as a marker for renal tubular damage, with a Podocalyxin rELISA Kit (EXOCELL) and a Rat TIM-1/KIM-1/HAVCR Qauntikine ELISA Kit (R&D Systems), respectively.

Compound 1 (0.1, 0.3 and 1 mg/kg) was orally administered to 8-week-old male ZDF rats once per day, for 12 weeks. As a control group, ZDF rats were orally administered a solvent (0.5% methyl cellulose) once per day for 12 weeks (Vehicle group). Urine was collected for 24 hours at the 12th week of administration, and the urinary albumin concentration, urine Podocalyxyn concentration, urine KIM-1 concentration and urinary volume were measured. At the 13th week after initial administration (24 hours after the final administration), the renal XO activity was measured. The urinary albumin, Podocalyxyn and KIM-1 excretions per day were calculated from the measured results, and the value for each group was compared to examine the drug effect. As a result of the examination, in the group administered compound 1, 0.3 mg/kg and 1 mg/kg doses significantly reduced the urinary albumin excretion compared to the Vehicle group, (Table 5). Moreover, the group administered compound 1 reduced not only in KIM-1, which is a marker for renal tubular damage that has already been reported to be improved by XO inhibitors, but also in Podocalyxyn which is a marker for glomerular damage. These results suggested that compound 1 improved both renal tubular damage and glomerular damage (Table 6 and Table 7). Since renal XO produces reactive oxygen species and uric acid that are thought to contribute to pathology of diabetic nephropathy, the renal XO activity was measured to confirm the curative effect of compound 1 on diabetic nephropathy. As a result, in the group administered compound 1, the 0.3 mg/kg and 1 mg/kg doses significantly inhibited renal XO activity compared to the Vehicle group, even at the 13th week after initial administration (24 hours after the final administration) (Table 8), suggesting a curative effect for diabetic nephropathy.

**[Table 5]**

| Urinary albumin excretion in each group at 12th week after initial administration | | | | |
|---|---|---|---|---|
| Group name | Vehicle | Compound 1 0.1 mg/kg | Compound 1 0.3 mg/kg | Compound 1 1 mg/kg |
| Urinary albumin excretion (µg/Day) | 120.14 ±15.16 | 96.50 ±9.61 | 70.18 ±60.94* | 67.63 ±14.70* |

| | | | | |
|---|---|---|---|---|
| (Values are mean ±SE) *p < 0.05: Significant difference (vs Vehicle group) (Dunnett-type multiple comparison test) | | | | |

**[Table 6]**

| Urinary Podocalyxyn excretion in each group at 12th week after initial administration | | | | |
|---|---|---|---|---|
| Group name | Vehicle | Compound 1 0.1 mg/kg | Compound 1 0.3 mg/kg | Compound 1 1 mg/kg |
| Urinary Podocalyxyn excretion (µg/Day) | 37.2 ±4.5 | 32.5 ±3.3 | 22.3 ±2.9* | 20.6 ±4.4* |

| | | | | |
|---|---|---|---|---|
| (Values are mean ±SE) *p < 0.05: Significant difference (vs Vehicle group) (Dunnett-type multiple comparison test) | | | | |

**[Table 7]**

| Urinary KIM-1 excretion in each group at 12th week after initial administration | | | | |
|---|---|---|---|---|
| Group name | Vehicle | Compound 1 0.1 mg/kg | Compound 1 0.3 mg/kg | Compound 1 1 mg/kg |
| Urinary KIM-1 excretion (ng/Day) | 46.4 ±9.5 | 64.3 ±22.1 | 22.3 ±2.9 | 18.1 ±2.0 |

| | | | | |
|---|---|---|---|---|
| (Values are mean ±SE) | | | | |

**[Table 8]**

| Renal XO activity of each group 24 hours after administration at 13th week after initial administration | | | | |
|---|---|---|---|---|
| Group name | Vehicle | Compound 1 0.1 mg/kg | Compound 1 0.3 mg/kg | Compound 1 1 mg/kg |
| Renal XO activity (µU/mg protein) | 21134 ±997 | 22574 ±2214 | 8752 ±1812** | 6331 ±839** |

| | | | | |
|---|---|---|---|---|
| (Values are mean ±SE) **p < 0.01: Significant difference (vs Vehicle group) (Dunnett-type multiple comparison test) | | | | |

### INDUSTRIAL APPLICABILITY

Compounds represented by formula (I) and their pharmaceutically acceptable salts can be used as therapeutic drugs and prophylactic drugs for diabetic nephropathy.

## Claims

1. A therapeutic drug or prophylactic drug for diabetic nephropathy containing a compound represented by formula (I) or its pharmaceutically acceptable salt as an active ingredient. 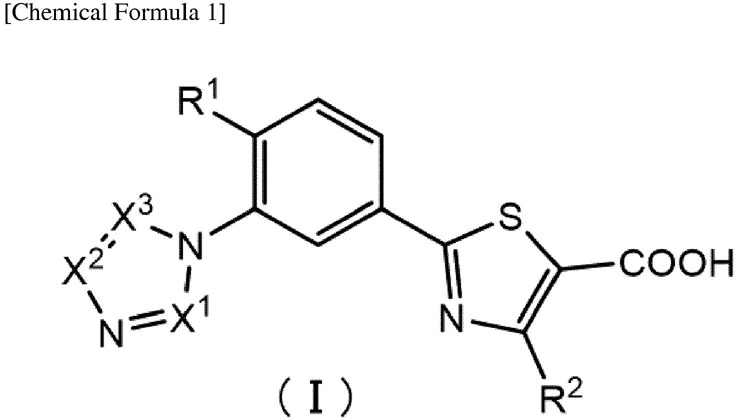 [wherein,
R¹ represents OR, NRR' that optionally forms a ring, or SR, where R and R' each independently represent a hydrogen atom, an alkyl group of 1 to 8 carbon atoms optionally substituted with one or more alkoxy groups of 1 to 8 carbon atoms, halogen atoms or hydroxyl groups, an aryl group optionally substituted with one or more alkyl groups of 1 to 8 carbon atoms, alkoxy groups of 1 to 8 carbon atoms or halogen atoms, or a heteroaryl group optionally substituted with one or more alkyl groups of 1 to 8 carbon atoms, alkoxy groups of 1 to 8 carbon atoms or halogen atoms.
R² represents a hydrogen atom or an alkyl group of 1 to 8 carbon atoms.
X¹ , X² and X³ are each independently CR³ or a nitrogen atom, or X¹ is CR³ or a nitrogen atom, and X² and X³ together form a benzene ring. R³ represents a hydrogen atom or an alkyl group of 1 to 8 carbon atoms.]

2. The therapeutic drug or prophylactic drug according to claim 1, wherein R¹ is OR.

3. The therapeutic drug or prophylactic drug according to claim 1, wherein R¹ is SR.

4. The therapeutic drug or prophylactic drug according to claim 1, wherein R¹ is NRR' that optionally forms a ring.

5. The therapeutic drug or prophylactic drug according to any one of claims 1 to 4, wherein R and R' are each independently an alkyl group of 1 to 8 carbon atoms optionally substituted with one or more alkoxy groups of 1 to 8 carbon atoms, halogen atoms or hydroxyl groups, or an aryl group optionally substituted with one or more alkyl groups of 1 to 8 carbon atoms, alkoxy groups of 1 to 8 carbon atoms or halogen atoms.

6. The therapeutic drug or prophylactic drug according to claim 5, wherein R and R' are each independently an alkyl group of 1 to 8 carbon atoms optionally substituted with one or more alkoxy groups of 1 to 8 carbon atoms, halogen atoms or hydroxyl groups.

7. The therapeutic drug or prophylactic drug according to claim 6, wherein R¹ is OR or SR, and R is an isopropyl, isobutyl or neopentyl group.

8. The therapeutic drug or prophylactic drug according to any one of claims 1 to 7, wherein R² is a hydrogen atom or an alkyl group of 1 to 3 carbon atoms.

9. The therapeutic drug or prophylactic drug according to claim 8, wherein R² is a methyl group.

10. The therapeutic drug or prophylactic drug according to any one of claims 1 to 9, wherein X¹, X² and X³ are each independently CR³ or a nitrogen atom.

11. The therapeutic drug or prophylactic drug according to claim 10, wherein X¹ is a nitrogen atom, X² is CR³ or a nitrogen atom, and X³ is CR³.

12. The therapeutic drug or prophylactic drug according to any one of claims 1 to 11, wherein R³ is a hydrogen atom.

13. The therapeutic drug or prophylactic drug according to claim 1, wherein the compound represented by formula (I) is a compound selected from the following group, or a pharmaceutically acceptable salt of the same.

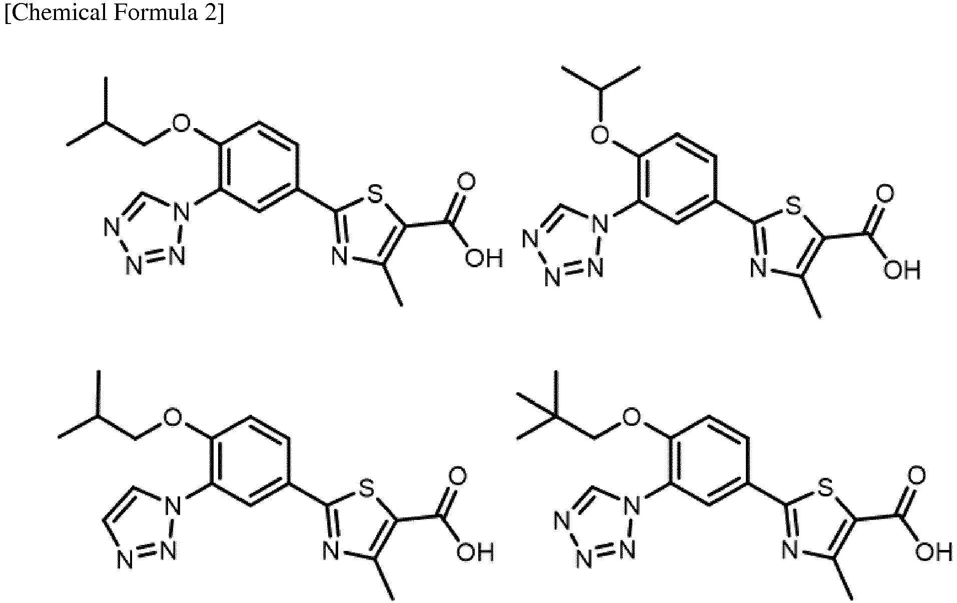

14. The therapeutic drug or prophylactic drug according to any one of claims 1 to 13, wherein the diabetic nephropathy is type II diabetic nephropathy.
